# FASCICULE DE BREVET EUROPEEN

(11) **EP 2 198 874 B1**
(45) Date de publication et mention de la délivrance du brevet: **23.08.2017**
(21) Numéro de dépôt: 08022273.0
(22) Date de dépôt: 22.12.2008
(51) Int. Cl.: A61K 36/898, A61K 36/03, A61K 35/02, A61P 25/24

(54) **Compositions thérapeutiques destinées au traitement de la dépression**
Therapeutische Zusammensetzungen für die Behandlung von Depressionen
Therapeutic compositions designed for treating depression

(43) Date de publication de la demande: 23.06.2010
(73) Titulaire: Patrinove, 69006 Lyon (FR)
(72) Inventeur: Serrar, Mostafa, 69720 Saint Bonnet de Mure (FR); Gutierrez, Gilles, 1361 Qrendi Qrd (MT)
(74) Mandataire: Tripoz, Inès

(56) Documents cités:
- WO-A-2007/085946
- FR-A- 2 774 292
- US-A- 4 438 104
- "Vanilla tahitensis"[Online] pages 1-2, XP002532112 INTERNET Extrait de l'Internet: URL:http://fr.wikipedia.org/wiki/Vanilla_t ahitensis>
- EHLERS DOROTHEA ET AL: "Analysis of CO-2 vanilla extracts by high performance liquid chromatography. Comparison with the usual alcoholic vanilla extracts" ZEITSCHRIFT FUER LEBENSMITTEL-UNTERSUCHUNG UND -FORSCHUNG, vol. 197, no. 6, 1993, pages 550-557, XP008107298 ISSN: 0044-3026
- LONGHURST IAN: "The Identity of Pliny's Flos Salis and Roman Perfume = L'identité de la Flos Salis de Pline l'Ancien et du parfum romain" AMBIX, vol. 54, no. 3, 2007, pages 299-302, XP008107351
- "Fleur de sel"[Online] pages 1-2, XP002532113 INTERNET Extrait de l'Internet: URL:http://fr.wikipedia.org/wiki/Fleur_de_ sel> -& "Histoire du salin - Technique d'explotation du salin - La fleur de sel du Camargue"[Online] pages 1-2, XP002532114 INTERNET Extrait de l'Internet: URL:http://www.ot-aiguesmortes.fr/FR/Salin .htm> -& "Dunaliella salina"[Online] pages 1-2, XP002532115 INTERNET Extrait de l'Internet: URL:http://fr.wikipedia.org/wiki/Dunaliell a_salina>
- GOLD M S ET AL: "Antimanic, antidepressant, and antipanic effects of opiates: Clinical, neuroanatomical, and biochemical evidence" ANNALS OF THE NEW YORK ACADEMY OF SCIENCES 1982 US, vol. Vol. 398, 1982, pages 140-150, XP002532824 ISSN: 0077-8923
- BYTHROW ET AL: "Vanilla as a Medicinal Plant", SEMINARS IN INTEGRATIVE MEDICINE, ELSEVIER, US, vol. 3, no. 4, 1 December 2005 (2005-12-01), pages 129-131, XP005533490, ISSN: 1543-1150, DOI: 10.1016/J.SIGM.2006.03.001

## Description

La présente invention se rapporte au domaine des nécessités de la vie et plus particulièrement au domaine de la thérapeutique.

Elle a plus précisément pour objet de nouvelles compositions thérapeutiques destinées à traiter les états dépressifs et les manifestations de déprime sous toutes ses formes.
En effet, aucune thérapeutique efficace de la dépression, ou « déprime », dans le contexte de la nutraceutique aussi bien que la pharmacie thérapeutique contemporaine n'utilise à ce jour l'activité de neuro-hormones telles que la béta-endorphine et la met-enképhaline, en raison de leur durée de vie trop courte.

La présente invention se caractérise donc par le fait d'associer des substances d'origine naturelle qui agissent sur l'humeur en utilisant l'activité de neuro-hormones. Ces substances amplificatrices de l'expression de la béta-endorphine sont associées à une ou des substances naturelles visant à augmenter la durée de vie de la béta-endorphine, en inhibant la dégradation des neuro-hormones.

### Arrière-plan technologique :

A ce jour, le traitement médicamenteux de la dépression repose sur la pharmacologie et la psychologie. Les drogues les plus utilisées sont essentiellement des substances visant à préserver l'activité des catécholamines et de la sérotonine. L'augmentation de la durée de vie de ces substances a pour but d'augmenter la concentration des neurotransmetteurs dans la fente synaptique. Pour ce faire, il existe des substances qui inhibent leur dégradation par la voie de la monoamine oxydase (IMAO), soit en ralentissant la recapture du neuromédiateur de manière sélective ou non : antidépresseurs tricycliques, inhibiteurs sélectifs de la recapture de la noradrénaline ou de la sérotonine, inhibiteurs ubiquitaires de la recapture de la sérotonine et de la noradrénaline.

Dans les temps anciens, Pline l'Ancien (Caius Plinius Secundus) avait rapporté l'existence de corps gras aux curieuses propriétés (*Oleum Flos salis*). Il avait noté que la fleur de sel (*flos salis*) ne présentait pas uniquement des propriétés gustatives.

Dans son « Histoire naturelle », Pline l'Ancien mentionne la fleur de sel (flos salis) comme étant le meilleur type de sel (volume 31 sur 37 volumes au total, chapitre XLII). Cette fleur de sel produit une sorte d'huile aussi surprenant que cela puisse paraître (Optimo ex eo, quod olei quamdam pinguitudinem reddit. Est enim etiam in sale pinguitudo, quod miremur). Il y a de la graisse même dans le sel ! S'exclame-t-il. Pline l'Ancien ajoute qu'elle n'a pas de pouvoir nutritif mais qu'elle est relâchante, (décontractante), stimulante et pouvant porter remède à la lassitude (psychostimulante) *solvit in vino et aqua, acopis et smegmatis utilis.* Toutefois, la consommation excessive de fleur de sel apportera bien d'autres inconvénients par son apport massif en sodium.

### La fleur de sel :

La fleur de sel est délicatement cueillie, selon une méthode artisanale et millénaire, à la surface de l'eau des oeillets par les paludiers. Non broyés, non lavés, ses cristaux préservent leur richesse naturelle en oligo-éléments. La saveur incomparable de la fleur de sel est particulièrement prisée par les gourmets et les chefs cuisiniers dont l'imagination en matière de composition culinaire est décuplée par cette denrée.

Les auteurs précédents ont pu démontrer que le biofilm déposé avec les cristaux de sel provient de la vie animale et végétale qui se met sous une forme de résistance pour survivre non seulement à l'augmentation colossale de la pression osmotique mais aussi à des conditions drastiques de sècheresse et parfois de température. La forme de résistance la plus appropriée est l'oeuf (zygote) issu de la fécondation de gamètes femelles et mâles (haploïdes). L'appariement sexuel ne peut se faire sans un échange de messages entre les deux diploïdes qui devront s'appareiller. Au moins l'une de ces substances messagères échangées est reconnue par les cellules épithéliales des mammifères. Il existe un lien entre la production de la β-endorphine issue du clivage de la pro-opiomélanocortine et l'attrait et le plaisir sexuels. Pour les formes de vie unicellulaire, la reconnaissance des partenaires pour une reproduction sexuée passe par l'échange de signaux apparentés à la production de la P-OMC chez les mammifères.

Les demandeurs ont démontré que la libération de la béta-endorphine et de son précurseur la pro-opiomélanocortine en culture cellulaire était amplifiée lorsque des cellules épithéliales telles que les kératinocytes sont cultivées en présence de l'extrait huileux de fleur de sel solubilisé qui est ajouté au milieu de culture.

Les effets de l'extrait huileux de *flos salis* sur la synthèse de la bêta endorphine sont mesurés par immunofluorescence indirecte.

**Essais en triplicate.**

| | | D.O. moyenne sur la synthèse de β-endorphine. | Écart type |
|---|---|---|---|
| | Contrôle | 0,181 | 0,014 |
| E. Flos salis | 0,25µg/ml | 0,182 | 0,019 |
| E. Flos salis | 0,5µg/ml | 0,204 | 0,021 |
| E. Flos salis | 1 µg/ml | 0,296 | 0,014 |
| E. Flos salis | 5µg/ml | 0,298 | 0,025 |

La présence de la béta-endorphine est notée pendant un laps de temps relativement bref en dépit du fait que les cellules soient isolées du métabolisme général.

Il est connu que les peptides épithéliaux sont souvent emmagasinés sous la lame basale de l'épithélium dans l'héparane sulfate. Les demandeurs ont montré par ailleurs (brevet français 2.896.692) que l'extrait de la padine (phéophycées : *Padina pavonica, japonica* etc.) amplifiait considérablement la production des glycos aminoglycanes comme l'héparane sulfate (HS) lorsqu'il était ajouté à une culture de fibroblastes.

Le brevet français 2.774.292 rapporte des compositions pharmaceutiques, cosmétiques et/ou alimentaires destinées à stimuler ou à amplifier la synthèse de la pro-opiomélanocortine et de ses dérivées comme la bêta-endorphine par les cellules épithéliales de mammifères avec des extraits de Cyclotella (Diatomées).

Expression de la β-endorphine dans le milieu de culture dosage par ELISA.

| | T=0 | 1h | 2h | 3h | 4h | 8h |
|---|---|---|---|---|---|---|
| sans HS | 100% | 78% | 55% | 37% | 26% | 10% |
| avec HS | 100% | 100% | 87% | 81% | 79% | 66% |

Ces données montrent que la quantité restante de β-endorphine (βe) non dégradée s'exprime selon l'équation : Q βe [HS-] = 0,9644 e ^{-0,291,t}. Ainsi la moitié de la β-endorphine se dégrade en 2 h. La demi-vie (t ^{1/2}) de la β-endorphine est donc de 120 minutes alors qu'en présence d'héparane sulfate, dans les mêmes conditions la quantité de β-endorphine non dégradée s'exprime selon l'équation :
Q βe [HS-] = 0,9962 e^{-0,0542.t} En présence d'héparane sulfate la moitié de la β-endorphine se dégrade en 10 h. La demi-vie (t ½) de la β-endorphine en présence d'héparane sulfate est donc de 600 minutes.

### Importance de Vanilla tahitensis

L'utilisation de la Vanille comme plante médicinale était connue depuis les Aztèques. Pendant les années 1700 et 1800, 4 la vanille était présente dans la Pharmacopée Européenne et a été indiquée pour les fièvres, la mélancolie, l'hystérie et comme stimulant nerveux (J.B. Bythrow, 2005. Seminars in Integrative Medicine, 3(4): 129-131).

Il est connu et décrit dans les ouvrages de physiologie humaine que les catécholamines sont transformées en acide vanilmandélique après le métabolisme enzymatique par la mono-amine-oxydase. D'autre part, la vanille contient une substance odoriférante connue sous le nom de vanilline et ses esters. L'acide vanilmandélique présente une très grande homologie de structure avec la vanilline. Une grande partie de leur formule est superposable. Les demandeurs ont constaté aussi que l'adjonction d'extrait alcoolique de vanille à une culture de kératinocytes traitée préalablement par l'extrait de fleur de sel présentait une prolongation considérable de la demi-vie des peptides des neuro-hormones comme la béta-endorphine par rétroaction sur les réactions enzymatiques. Les résultats du tableau ci-dessous mettent en évidence une synergie entre les extraits naturels de Flos Salis (FS) et l'extrait de Vanilla tahitensis (EVT) en raison de l'interaction de la vanilline naturelle avec le métabolisme des catécholamines conduisant à l'acide vanilmandélique.

| | Signal | écart type |
|---|---|---|
| Contrôle | 0,1280 | 0,005 |
| *Flos salis* | 0,1960 | 0,006 |
| EVT | 0,1440 | 0,009 |
| FS + VT | 0,2600 | 0,009 |

La fleur de sel (*Flos salis* : FS) trouve une définition dans la littérature, selon l'encyclopédie Wikipédia : La fleur de sel est la mince couche de cristaux blancs qui se forme à la surface des marais salants, en général par l'action évaporatrice du vent. Elle est utilisée en cuisine à la place du sel pour assaisonner les plats.

La fleur de sel connaît depuis quelques années une certaine renommée auprès des gastronomes.

Très blanche aux fins cristaux neigeux, parfumés d'un léger goût de violette, elle agrémente la table en relevant le goût des plats les plus délicats. La fleur de sel se dissout très rapidement, ce qui lui confère un haut pouvoir pénétrant dans les aliments qu'elle assaisonne. Ceci est la raison pour laquelle, en cuisine, il est conseillé de l'ajouter en fin de cuisson.

Sa couleur parfois rosée ou saumon est due à la prolifération de *Dunaliella salina*, une algue rouge microscopique, qui lui procure une agréable odeur de violette. Riche en magnésium ainsi qu'en oligo-éléments, son goût est réputé beaucoup plus fin et délicat que le sel de table.

C'est pourquoi les demandeurs ont réalisé, et c'est ce qui constitue l'invention, un mélange de trois substances, Vanilla, Padina pavonica et extrait huileux de fleur de sel, ledit mélange, étant présenté de préférence sous forme de comprimés, de gélules, de boissons, ou toute autre forme galénique permettant une délivrance systémique ou locale de cette composition. L'expression Vanilla désigne aussi bien la poudre de plante qu'un extrait de plante obtenu par lixiviation ou macération de la plante. L'expression Padina pavonica désigne également la poudre séchée de cette algue qu'un extrait obtenu par action d'un solvant.

Les compositions selon l'invention conviennent pour le traitement de la lassitude, de la déprime, des états de mal être et même de la dépression. En effet, il faut se rappeler que les neurohormones comme la β-endorphine jouent un rôle essentiel dans le bien être. Ces hormones sont essentiellement élaborées au niveau des tissus épithéliaux. Lors du vieillissement les humains voient le volume kératinocytaire épithélial diminuer considérablement. Il s'ensuit qu'un certain nombre d'individus ne produiront plus la bonne quantité de neurohormones. Selon la sévérité des signes, les doses d'extrait alcoolique de Vanilla varieront de 1 à 100 mg, et de préférence de 2 à 50 mg par prise unitaire ; celles d'extrait de *Padina pavonica* varieront de 50 à 500 mg (en équivalent de plantes sèches) et de préférence de 100 à 200 mg par prise unitaire ; de même les quantités d'extrait huileux de fleur de sel varieront de 1 à 100 mg et de préférence de 5 à 50 mg par prise unitaire.

Les compositions pharmaceutiques ou alimentaires selon l'invention sont destinées à la voie orale chez l'homme ou l'animal. Elles se présentent sous l'une des formes qui conviennent pour l'administration par cette voie, telles que gélules, comprimés, poudres aromatisées ou non, dragées, solutions, suspensions, émulsions, potions ou sirop.

Les excipients ou les véhicules sont ceux utilisés habituellement dans les formes orales.
La consommation ou posologie sera de préférence de 1 à 6 gélules ou comprimés par jour pendant une période d'au moins 15 jours.

Les exemples suivants illustrent l'invention sans toutefois la limiter.

### Exemple 1 :

Fabrication de comprimés correspondant à la formulation ci-dessous.

Formule préparée avec des extraits pesés en équivalent de poids de plantes sèches :

| | |
|---|---|
| Extrait alcoolique de *Vanilla tahitensis* | 50 mg |
| Extrait de *Padina pavonica* | 100 mg |
| Extrait alcoolique de fleur de sel (*Flos salis)* | 5 mg |
| Cellulose microcristalline QSP un comprimé de | 250 mg |

### Exemple 2 :

Fabrication de gélules n°1 correspondant à la formulation ci-dessous.

Formule préparée avec des extraits pesés en équivalent de poids de plantes sèches :

| | |
|---|---|
| Extrait alcoolique de *Vanilla tahitensis* | 2 mg |
| Extrait de *Padina pavonica* | 200 mg |
| Extrait alcoolique de fleur de sel (*Flos salis*) | 50 mg |
| Cellulose microcristalline QSP une gélule terminée à | 250 mg |

### Exemple 3 :

Fabrication de boisson correspondant à la formulation ci-dessous.

Formule préparée avec des extraits pesés en équivalent de poids de plantes sèches :

| | | |
|---|---|---|
| Extrait alcoolique de *Vanilla tahitensis* | | 120 mg |
| Extrait de *Padina pavonica* | | 1200 mg |
| Extrait alcoolique de fleur de sel (*Flos salis*) | | 300 mg |
| Glycérine | | 150 ml |
| Eau aromatisée | QSP | 300 ml |

La consommation ou posologie sera de préférence d'un flacon répartie en prises égales quotidiennes de 2 ml pendant 15 jours.

### Exemple 4 :

Mise en évidence de l'effet sur la β-endorphine :
Le choix d'étudier la cinétique de dégradation de la β-endorphine dans le surnageant de culture a été fait afin de reproduire plus ou moins exactement les propriétés physiologiques en milieu extracellulaire de l'organisme dans lequel le neuromédiateur est naturellement libéré. Ainsi, dans les milieux extracellulaires « in-vivo » comme « in-vitro » il y a de nombreuses autres molécules biologiquement actives et notamment des enzymes de dégradation qui peuvent interférer dans la stabilité et l'activité de la β-endorphine.

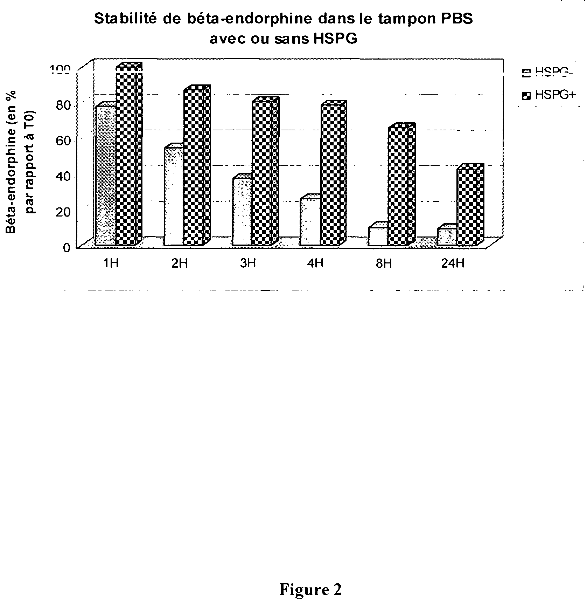

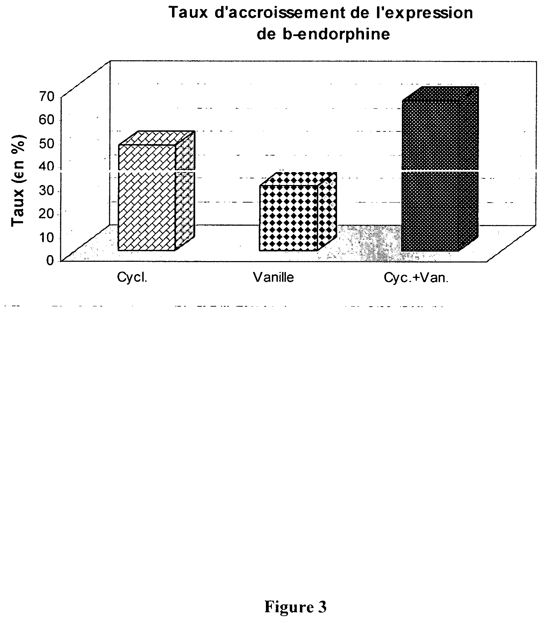

Ces résultats sont mis en évidence dans les figures 2 et 3 ci-après annexées.

### Système neuro-cutané :

Le système nerveux et le tissu cutané sont très étroitement liés à la fois par leur origine embryonnaire (le système nerveux et la peau proviennent tous les deux du même tissu, l'ectoderme) et par l'existence de connexions cutanéo-nerveuses distales, les prolongements dendritiques des cellules neuronales étant présentes aussi bien dans le derme que dans l'épiderme. Les cellules nerveuses ont de véritables contacts avec les cellules cutanées et notamment avec les kératinocytes.

### Neuromédiateurs cutanés :

II a été mis en évidence au niveau de la peau humaine la présence de nombreux neuromédiateurs parmi lesquels on peut citer la substance P, le neuropeptide Y, le CGRP (calcitonin gene-related peptide), le VIP (vasoactive intestinal peptide) et les POMC (proopimelanocortines). Ces neuromédiateurs sont libérés bien évidemment par les cellules nerveuses elles-mêmes. Ainsi, les populations kératinocytaires et fibroblastiques sont capables de synthétiser les POMC dans des conditions normales en quantités non négligeables.

### β-endorphine :

Il s'agit d'un neuromédiateur et plus exactement d'une neurohormone appartenant à la famille des mélanocortines qui inclut aussi d'autres molécules comme ACTH, MSH et Metenképhaline.

Ces neuropeptides proviennent tous de la même protéine qui est la proopiomelanocortine. Cette dernière va subir un clivage protéolytique pour se scinder en plusieurs petites molécules peptidiques (citées ci-dessus) biologiquement actives. Toutefois, la β-endorphine reste le représentant principal de cette famille surtout pour son rôle physiologique important dans l'organisme. En effet, la β-endorphine est connue pour ses effets divers et notamment ses effets analgésiques et anti-inflammatoires provoquant ainsi des sensations de confort, de bien-être, de plaisir voire d'euphorie. Il est évident que la molécule de β-endorphine puisse présenter un grand intérêt et ceci non seulement pour ses propriétés évoquées précédemment, mais aussi parce qu'elle peut être synthétisée sur place par des cellules de la peau, cellules dont l'activité de synthèse peut être modulée ouvrant ainsi la voie à une cosmétologie active vers l'obtention du confort, du bien-être, et du plaisir.

### Stabilisation de l'activité biologique de β-endorphine :

Comme toute molécule de nature peptidique, la β-endorphine est très instable dans les milieux biologiques avec une durée de vie très courte à cause de la présence dans le milieu intercellulaire d'enzymes de dégradation, regroupées sous l'appellation générale de protéases. En effet, aussitôt synthétisées par les cellules et libérées dans le milieu extracellulaire, les molécules de β-endorphine sont aussitôt dégradées par ces protéases. Pour prolonger dans le temps les sensations de bien-être et de plaisir, il est donc obligatoire de maintenir stable ce type de peptide.

Les dosages de β-endorphine intra et extracellulaire montrent une stimulation importante de l'expression du neuropeptide par les cultures de kératinocytes après addition de l'extrait de Cyclotelle seule ou associée à un extrait de Vanilla tahitensis et ce, dès les premières 24 heures d'incubation.

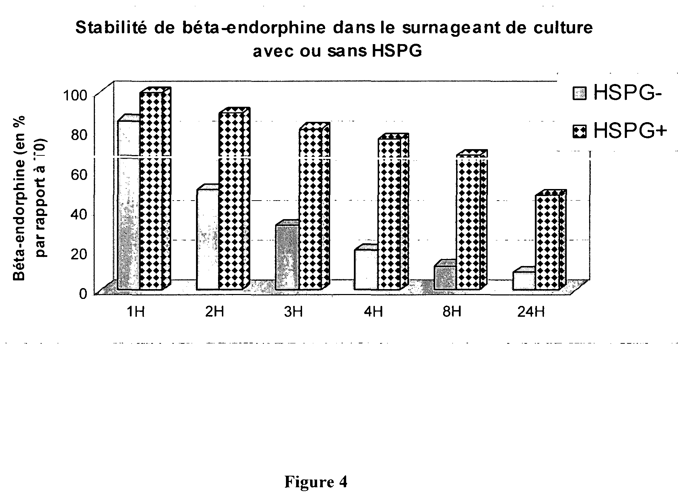

Par ailleurs, on note une induction significative de l'expression de β-endorphine par un extrait de Padina pavonica (EPP) seul à partir de 48 heures pour les kératinocytes. Cette induction est liée indirectement à l'effet réservoir de l'héparane-sulfate dont la synthèse est largement stimulée par un extrait de Padina pavonica, ce qui explique aussi l'efficacité plus importante de l'extrait de Cyclotelle lorsqu'il est associé à un extrait de Padina pavonica. En effet, comme un extrait de Padina pavonica augmente la synthèse des protéoglycanes et notamment de l'héparane-sulfate, on assiste à une fixation en grand nombre des molécules de β-endorphine par cette matrice extracellulaire riche en protéoglycanes qui va les accumuler au fil du temps tout en préservant leur activité biologique comme il est montré aux figures 4 et 5.

### Comment faire durer le plaisir ? :

L'effet clinique insuffisant observé avec seulement l'extrait de Cyclotelle est dû à la disparition rapide par dégradation extracellulaire de β-endorphine. On a donc recherché un procédé capable de stabiliser la molécule très rapidement au moment de sa synthèse. Il a été trouvé qu'une quantité importante d'héparane-sulfate -protéoglycane- localement, permet de stabiliser la molécule. En effet, ce protéoglycane est connu pour développer une forte affinité vis-à-vis de nombreuses molécules physiologiques. Les molécules liées à l'héparane-sulfate deviennent beaucoup plus stables et plus actives. Pour induire la stabilité recherchée il est apparu judicieux de stimuler la synthèse de l'héparane-sulfate en rajoutant à l'extrait de Cyclotelle un autre composant comme l'extrait de Padina pavonica dont on connaît l'effet stimulant sur l'expression des protéoglycanes et notamment de l'héparane-sulfate.

Par ailleurs, il est à noter que dans le tissu cutané, l'héparane-sulfate est localisé surtout au niveau de la membrane basale et est synthétisé aussi bien par les kératinocytes (cellules épidermiques) que par les fibroblastes (cellules dermiques). La présence de ce protéoglycane en quantités suffisantes va donc permettre la fixation et par conséquent la stabilisation d'un plus grand nombre de molécules de β-endorphine en augmentant ainsi leur biodisponibilité. Le rôle réservoir que joue l'héparane-sulfate pour les molécules de β-endorphine est d'une importance capitale pour le maintien de leur activité et pour la prolongation de l'effet clinique désiré.

### Détermination de l'effet de la Padine sur la synthèse de β-endorphine :

### Méthode :

On mesure l'effet de l'extrait de Padine (EPP) qui est un stimulant de l'expression des protéoglycanes, sur la cinétique de synthèse de la β-endorphine par les kératinocytes et les fibroblastes en présence d'extrait de Cyclotelle.

### Protocole :

### Traitement des cellules :

Les kératinocytes ou les fibroblastes sont ensemencés à une densité de 50.000 cellules par puits (microplaques 96 puits) dans 200 µl de milieu de culture. Après une nuit d'incubation, les surnageants sont éliminés, puis remplacés par le même volume de milieu neuf seul (contrôle) ou contenant :
Soit de l'extrait de Cyclotelle
Soit l'extrait de Padine
Soit l'extrait de Cyclotelle + un extrait de Padina pavonica

Les cellules ainsi traitées sont incubées pendant 24 heures, 48 heures, 72 heures et 7 jours. Les milieux de culture et les traitements sont renouvelés tous les 2 jours.

A la fin de chaque intervalle de temps, les surnageants sont éliminés et les tapis cellulaires sont rincés 3 X 5 dans 300 µl du tampon PBS, puis fixés dans un volume de 50 µl d'une solution à 4 % de paraformaldéhyde contenant 0.01 % de Triton X-100. Après 30 minutes de fixation à 4°C, les tapis cellulaires sont rincés plusieurs fois dans le tampon PBS suivi immédiatement du marquage de la β-endorphine à l'aide de la technique d'immunofluorescence indirecte.

### Mesure de la fluorescence :

A la fin de la réaction d'immunofluorescence, les cellules sont lysées grâce à un tampon de lyse permettant la mise en solution de la fluorescéine. Les surnageants fluorescents ainsi obtenus sont lus au spectrofluorimètre (Fluroskan) à une longueur d'onde d'excitation de 485 nm et à une longueur d'onde d'émission de 518 nm. Les valeurs obtenues sont des valeurs arbitraires mais proportionnelles à la quantité de β-endorphine marquée.

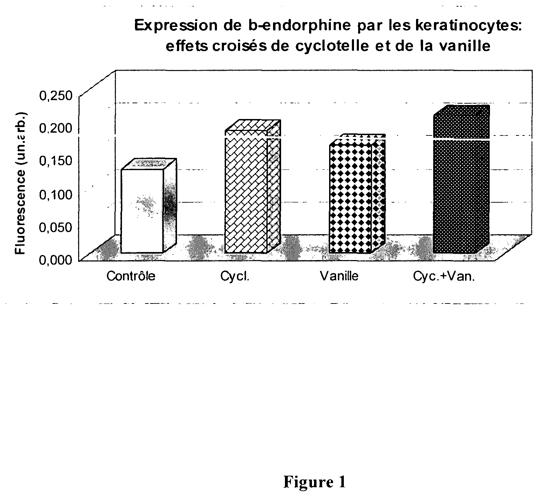

Les valeurs obtenues sont rassemblées dans les tableaux 1 et 2 ci-après et dans les figures 1 et 3 ci-après annexées.

**Tableau 1**

| *Effets de Cyclotella sur la synthèse de béta-endorphine :* | | | | | |
|---|---|---|---|---|---|
| | Value-1 | Value-2 | Value-3 | Average | S.D |
| Control | 0,183 | 0,167 | 0,194 | **0,181** | 0,014 |
| Cycl: 5µg/ml | 0,286 | 0,318 | 0,335 | **0,313** | 0,025 |
| Cycl: 1µg/ml | 0,296 | 0,277 | 0,304 | **0,292** | 0,014 |
| Cycl: 0,5µg/ml | 0,204 | 0,184 | 0,226 | **0,205** | 0,021 |
| Cycl: 0,25µg/ml | 0,182 | 0,174 | 0,211 | **0,189** | 0,019 |

Les effets de l'extrait de Cyclotelle sur la synthèse de la β-endorphine sont bien proportionnels aux doses.

**Tableau II**

| *Effets de l'extrait de Vanilla sur l'expression de béta-Endorphine en présence ou non d'extrait de Cyclotella (Fleur de sel) :* | | | | | |
|---|---|---|---|---|---|
| | Valeur 1 | Valeur 2 | Valeur 3 | Moyenne | Ecart-type |
| Contrôle | 0,134 | 0,124 | 0,127 | **0,128** | 0,005 |
| Cyclotella | 0,179 | 0,191 | 0,188 | **0,186** | 0,006 |
| Extrait de Vanilla | 0,174 | 0,161 | 0,158 | **0,164** | 0,009 |
| Cyc.+Vanilla | 0,22 | 0,201 | 0,208 | **0,210** | 0,010 |

L'association Cyclotella + extrait de Vanilla renforce sensiblement l'expression de β-endorphine comme montré également à la Figure 1.

**Effets de l'extrait de Fleur de sel (Cyclotelle) sur la synthèse de l'héparane-sulfate en l'absence ou en présence d'extrait de Vanilla :**

| | Taux d'accroissement |
|---|---|
| Cyclotelle | 45 |
| Vanilla | 28 |
| Cycl.+Vanilla | 64 |

L'effet de l'association Fleur de sel et extrait de Vanilla tahitensis est très net.

**Effet de l'extrait de Padina pavonica (EPP) sur la synthèse de l'héparane-sulfate :**

| | Taux d'accroissement de HS |
|---|---|
| EPP: 1µg/ml | 32% |
| EPP: 2µg/ml | 70% |
| EPP: 5µg/ml | 125% |
| EPP: 10µg/ml | 150% |

L'effet de l'extrait de Padina pavonica est considérable.

**Cinétique de dégradation de béta-Endorphine en présence ou en l'absence d'héparane-sulfate :**

| | 0H | 1H | 2H | 3H | 4H | 8H |
|---|---|---|---|---|---|---|
| Sans HSPG | 0,41 | 0,32 | 0,225 | 0,153 | 0,107 | 0,042 |
| Avec HSPG | 0,393 | 0,4 | 0,343 | 0,318 | 0,31 | 0,26 |

La dégradation de la β-endorphine en présence d'héparane-sulfate se trouve considérablement ralentie.
Ces données sont représentées sous forme de diagramme à la figure 2.

**Stabilité de la béta-Endorphine dans le surnageant de culture en présence ou en l'absence d'héparane-sulfate (HSPG) :**

| | 0H | 1H | 2H | 3H | 4H | 8H |
|---|---|---|---|---|---|---|
| Sans HSPG | 0,507 | 0,434 | 0,258 | 0,167 | 0,103 | 0,062 |
| Avec HSPG | 0,577 | 0,584 | 0,516 | 0,47 | 0,441 | 0,392 |

Ces données sont représentées sous forme d'un diagramme à la figure 4.

## Revendications

1. Compositions diététiques et/ou pharmaceutiques pour la voie orale, **caractérisées en ce qu'**elles renferment à titre de principes actifs un extrait de Vanilla tahitensis, un extrait de Padina pavonica et un extrait de Fleur de sel (flos salis), destinées à la consommation par voie orale, en mélange ou en association avec un ou plusieurs excipients acceptables la voie orale.

2. Compositions selon la revendication 1, **caractérisées en ce que** l'extrait de Padina pavonica est un extrait alcoolique ou acétonique de ladite algue.

3. Compositions alimentaires selon l'une des revendications précédentes, **caractérisées en ce que** l'extrait de Vanilla tahitensis est un extrait alcoolique de Vanilla tahitensis.

4. Compositions selon l'une des revendications précédentes, **caractérisées en ce que** l'extrait de fleur de sel est un extrait alcoolique ou huileux.

5. Compositions selon l'une des revendications précédentes, **caractérisées en ce que** la quantité d'extrait de Vanilla tahitensis présente dans les compositions est comprise entre 1 et 100 mg par prise unitaire.

6. Compositions selon l'une des revendications précédentes, **caractérisées en ce que** la quantité d'extrait de Padina pavonica présente dans les compositions, est comprise entre 50 et 500 mg par prise unitaire.

7. Compositions selon l'une des revendications précédentes, **caractérisées en ce que** la quantité d'extrait de fleur de sel présente dans les compositions est comprise entre 1 et 100 mg par prise unitaire.

8. Compositions selon l'une des revendications précédentes, **caractérisées en ce que** le ou les excipients sont choisis parmi ceux qui conviennent pour l'administration de formes liquides ou de gel, destinés à la voie orale.

9. Compositions selon l'une des revendications précédentes, **caractérisées en ce qu'**elles sont présentées sous forme de comprimés, de dragées, de gélules, de sachets de poudre, de potions, d'émulsions ou de sirops.

## Patentansprüche

1. Orale diätetische und/oder pharmazeutische Zusammensetzungen, **dadurch gekennzeichnet, dass** sie als Wirkstoffe einen Extrakt von Vanilla tahitensis, einen Extrakt von Padina pavonica und einen Extrakt von Fleur de Sel (Flos salis) umfassen, für die oral Einnahme in Kombination oder zusammen mit einem oder mehreren oral akzeptablen Trägern.

2. Zusammensetzung nach Anspruch 1, **dadurch gekennzeichnet, dass** der Extrakt von Padina pavonica ein alkoholischer oder ein azetonischer Extrakt dieser Alge ist.

3. Lebensmittelzusammensetzungen nach einem der vorangehenden Ansprüche, **dadurch gekennzeichnet, dass** der Extrakt von Vanilla tahitensis ein alkoholischer Extrakt von Vanilla tahitensis ist.

4. Zusammensetzungen nach einem der vorangehenden Ansprüche, **dadurch gekennzeichnet, dass** der Extrakt von Fleur de Sel ein alkoholischer oder öliger Extrakt ist.

5. Zusammensetzungen nach einem der vorangehenden Ansprüche, **dadurch gekennzeichnet, dass** die Menge an in den Zusammensetzungen enthaltenem Extrakt von Vanilla tahitensis zwischen 1 und 100 mg pro Dosiereinheit beträgt.

6. Zusammensetzungen nach einem der vorangehenden Ansprüche, **dadurch gekennzeichnet, dass** die Menge an in den Zusammensetzungen enthaltenem Extrakt von Padina pavonica zwischen 50 und 500 mg pro Dosiereinheit beträgt.

7. Zusammensetzungen nach einem der vorangehenden Ansprüche, **dadurch gekennzeichnet, dass** die Menge an in den Zusammensetzungen enthaltenem Extrakt von Fleur de Sel zwischen 1 und 100 mg pro Dosiereinheit beträgt.

8. Zusammensetzungen nach einem der vorangehenden Ansprüche, **dadurch gekennzeichnet, dass** der oder die Träger ausgewählt ist oder ausgewählt sind aus jenen, die für die orale Verabreichung als Flüssigkeiten oder Gele geeignet sind.

9. Zusammensetzungen nach einem der vorangehenden Ansprüche, **dadurch gekennzeichnet, dass** sie in Form von Tabletten, Dragées, Kapseln, Pulverpäckchen, Tränken, Emulsionen oder Sirupe vorliegen.

## Claims

1. Dietary and/or pharmaceutical compositions for oral administration, **characterized in that** they contain a Vanilla tahitensis extract, a Padina pavonica extract and a Fleur de sel (flos salis) extract as active ingredients, intended for oral consumption, in a mixture or in combination with one or more acceptable oral excipients.

2. Compositions according to Claim 1, **characterized in that** the Padina pavonica extract is an alcoholic or acetonic extract of said alga.

3. Food compositions according to one of the preceding Claims, **characterized in that** the Vanilla tahitensis extract is an alcoholic extract of Vanilla tahitensis.

4. Compositions according to one of the preceding Claims, **characterized in that** the Fleur de sel extract is an alcoholic or oily extract.

5. Compositions according to one of the preceding Claims, **characterized in that** the amount of Vanilla tahitensis extract that is present in the compositions is between 1 and 100 mg per unit dosage.

6. Compositions according to one of the preceding Claims, **characterized in that** the amount of Padina pavonica extract that is present in the compositions is between 50 and 500 mg per unit dosage.

7. Compositions according to one of the preceding Claims, **characterized in that** the amount of Fleur de sel extract that is present in the compositions is between 1 and 100 mg per unit dosage.

8. Compositions according to one of the preceding Claims, **characterized in that** the excipient, or the excipients is/are selected from those that are suitable for the administration of liquid or gel forms, intended for oral administration.

9. Compositions according to one of the preceding Claims, **characterized in that** they are presented in the form of tablets, lozenges, capsules, powder packets, potions, emulsions or syrups.
